# NEUE EUROPÄISCHE PATENTSCHRIFT

(11) **EP 1 670 371 B2**
(45) Veröffentlichungstag und Bekanntmachung des Hinweises auf die Entscheidung über den Einspruch: **08.06.2011**
(45) Hinweis auf die Patenterteilung: 10.12.2008
(21) Anmeldenummer: 04764428.1
(22) Anmeldetag: 24.08.2004
(51) Int. Cl.: A61B 17/32, A61B 17/22

(54) **FÖRDEREINRICHTUNG FÜR STERILE MEDIEN**
TRANSPORT DEVICE FOR STERILE MEDIA
DISPOSITIF DE REFOULEMENT POUR MILIEUX STERILES

(30) Priorität: 30.09.2003 DE 10345382; 21.10.2003 DE 10348832
(43) Veröffentlichungstag der Anmeldung: 21.06.2006
(73) Patentinhaber: Erbe Elektromedizin GmbH, 72072 Tübingen (DE)
(72) Erfinder: HAGG, Martin, 72827 Wannweil (DE); KÜHNER, Ralf, 70567 Stuttgart (DE)
(74) Vertreter: Bohnenberger, Johannes
(86) Internationale Anmeldenummer: PCT/EP2004/009451
(87) Internationale Veröffentlichungsnummer: WO 2005/034777

(56) Entgegenhaltungen:
- EP-A- 0 212 728
- EP-A- 0 470 781
- WO-A-99/33510
- WO-A-2002/050197
- FR-A- 2 722 840
- US-A- 4 137 011
- US-A1- 2002 045 912

## Beschreibung

Die Erfindung betrifft eine Fördereinrichtung für sterile Fluide, z.B. Ringer-Lösung, wie sie in der Wasserstrahlchirurgie Verwendung findet.

In der Wasserstrahlchirurgie bestehen im Allgemeinen folgende Anforderungen:
- Die Sterilität des Fluids muss gewährleistet bleiben, da es nicht nur mit dem Körper eines Patienten in Kontakt gelangt sondern auch teilweise im Körper verbleibt;
- der geförderte Fluss bzw. der dafür erforderliche Druck muss im Wesentlichen konstant und reproduzierbar einstellbar sein, um einen reproduzierbaren Gewebeeffekt zu erzielen;
- die Bedienungsperson muss frei sein in der Dauer der Einzelaktivierung mit konstantem Fluss;
- fürdas Fluid sollten Behältnisse in unterschiedlichen Größen vorhanden sein, damit für einen bestimmten Einsatz die benötigte Menge vorgehalten werden kann, ohne dass einerseits das Behältnis während einer Anwendung ausgetauscht werden muss und andererseits ein zu großes Behältnis nur teilweise, entleert wird;
- die Aktivierung des Instrumentes muss im Wesentlichen verzögerungsfrei begonnen und beendet werden können, d.h., der Druckaufbau muss schnell erfolgen und ein Nachtropfen darf nicht auftreten;
- Applikatoren müssen unproblematisch auswechselbar sein.

Chirurgische Wasserstrahlschneideinrichtungen und dazu gehörige Systeme zum Druckaufbau sind an sich bekannt. Eine technische Lösung, das Arbeitsfluid vollkommen steril zu halten, da es mit Teilen des Pumpmechanismus gar nicht in Kontakt tritt, zeigt die DE 42 00 976 C2 auf. Bei dieser bekannten Lösung ist der Anwender allerdings an eine quantisierte Menge des Fluids gebunden, da dies in einer vorbereiteten Kartusche enthalten ist. Ist diese einmal geöffnet, kann der verbleibende Inhalt nicht weiterverwendet werden und zwar unabhängig davon, wie viel oder wenig in der aktuellen Operation entnommen wurde. Der verbliebene Rest muss entsorgt werden. Die maximale Größe des Reservoirs bestimmt bei der bekannten Anordnung maßgeblich die Gerätedimensionen. Wenn ein kleines Gerät gewünscht ist, kann ein Wechsel des Reservoirs während der Operation leicht nötig werden, was eine störende Unterbrechung im OP-Ablauf darstellt. Weiterhin muss das Reservoir insgesamt unter Druck gesetzt werden, so dass eine Abhängigkeit von den zur Verfügung gestellten Größen für das Reservoir besteht.

Sterilfilter, die eine nichtsterile Förderung zulassen, indem sie mögliche Keime, die aus einer nichtsterilen Fördereinrichtung in das Trennmedium eingebracht werden können, herausfiltern, haben verschiedene Nachteile. Zum einen wird der Wartungsaufwand wesentlich erhöht, da die Filter in definierten zeitlichen Abständen gewechselt werden müssen. Des Weiteren wirken Filter auf das Fließverhalten hemmend. Bei hohen Drücken eignen sich eingesetzte Sterilfilter nur bedingt, da sie beim Ein- und Ausschalten hohen Flussänderungen unterworfen sind und so auch den geforderten schnellen Druckaufbau behindern.

Aus der US 2002/0045912 A1 ist eine Einrichtung zur Blutprobennahme bekannt, wobei hierbei ein Fluidstrahl eingesetzt wird. Der Fluidstrahl dient dem Durchschneiden von Gewebe, um Blut entnehmen zu können. Die Einrichtung weist z. B. einen sich in einem Zylinder hin- und herbewegenden Kolben zum Erzeugen des Strahls und eine Fluidquelle auf. Ferner ist mindestens ein Ventil vorgesehen. Druck und Dauer des Strahls sind einstellbar.

Bei Pumpen für medizinische Zwecke ist es zwingend erforderlich, insbesondere ein absolut steriles Fluid an ein etwaiges Operationsgebiet heranzuführen. Eine Reinigung und Sterilisation des als integrale Einheit ausgebildeten Pumpensystems ist sehr aufwändig und kaum zu bewerkstelligen.

Aus der WO 99/33510 A1 ist ein Fluidstrahl-System und ein Verfahren zur Anwendung in der Herzchirurgie bekannt. Hierbei soll ein Hochdruck-Fluidstrahl zum Schneiden oder Bohren erzeugt werden, um Herzgewebe zu behandeln. Ein Pumpeneinsatz ist als "disposable unit" vorgesehen. Das austauschbare bzw. Einweg-Teil weist den Einlassbereich und den Auslassbereich für das Fluid mit entsprechenden Ventilen auf. Bei diesem Pumpensystem ist es allerdings schwierig, konstante Druckverhältnisse zu erzeugen, die eine sichere und einfache Anwendung der Anordnung gewährleisten würden.

Der Erfindung liegt die Aufgabe zu Grunde, eine Fördereinrichtung für sterile Fluide aufzuzeigen, welche die Anwendbarkeit im Operationssaal vereinfacht und verbessert.

Diese Aufgabe wird durch eine Fördereinrichtung nach den Ansprüchen 1 und 2 gelöst.

Bei der ersten Alternative weist die Pumpe mindestens eine erste und eine zweite Kolben-/Zylinder-Einheit oder dergleichen Pumpkammern auf, die vorzugsweise im Gegentakt derart steuerbar sind, dass der Ansaugzyklus in der ersten Pumpkammer kürzer ist als der Ausstoßzyklus in der zweiten Pumpkammer und umgekehrt. Dadurch wird mit nur zwei Pumpkammern gewährleistet, dass ein kontinuierlicher Fluss erzeugt wird.

Die Antriebseinrichtungen sind derart ausgebildet, dass sich die Ausstoßzyklen überlappen. Dadurch wird ein besonders gleichmäßiger Fluss ohne Zuhilfenahme von Speichern oder dergleichen gewährleistet.

Die Antriebseinrichtung ist vorzugsweise derart ausgebildet, dass das Medium dem Verbraucher mit einem im Wesentlichen konstanten Druck und damit konstanter Fördermenge zugeführt wird. Dies erleichtert die Anwendbarkeit erheblich.

Die Pumpe ist mit der Antriebseinrichtung lösbar verbunden. Dadurch kann ein "steriler Vorrichtungsteil" von einem nur mit größtem Aufwand steril zu machenden "unsterilen Vorrichtungsteil" getrennt werden.

Die Pumpe und/oder die Leitungseinrichtungen sind zusammen mit den Ventileinrichtungen als "disposable unit" ausgebildet. Für jede Anwendung wird also ein "Set" bestehend aus Pumpen, den Leitungseinrichtungen und den notwendigen Ventileinrichtungen einerseits mit dem Reservoir für das zu fördernde Medium, andererseits mit dem chirurgischen Instrument verbunden und dann an die Antriebseinrichtung angekoppelt. Nach Benutzung wird dieses "Set" komplett entsorgt. Auf diese Weise ist vollkommene.

Sterilität bei einfacher Handhabung zu gewährleisten, da das "Set" beim Hersteller in einfacher Weise sterilisiert werden kann.

Für die Antriebseinrichtungen sind verschiedene Alternativen möglich. Zum einen kann für jede Pumpkammer ein gesondert steuerbarer Antriebsmotor vorgesehen sein, so dass mit geringem Aufwand unter Zuhilfenahme einer entsprechenden elektronischen Steuerung (z.B. unter Verwendung von Schrittmotoren) das Geschwindigkeitsprofil für die einzelnen Pumpkammern beliebig eingestellt werden kann. Alternativ (ggf. aber auch zusätzlich) können die Antriebseinrichtungen Getriebeeinrichtungen mit einem mit dem Antriebsmotor verbundenen Getriebe und einem Getriebeausgang für jede Pumpkammer vorgesehen sein. Es lässt sich zwar während des Betriebes das Verhältnis von Ansaugzyklus zu Ausstoßzyklus nicht verändern, jedoch ist die Förderrate sehr leicht einstellbar. Der Aufwand hierfür ist ebenfalls relativ gering.

Nachfolgend werden bevorzugte Ausführungsformen der Erfindung an Hand von Abbildungen näher erläutert. Hierbei zeigen
- Fig. 1 eine schematisierte Darstellung einer ersten Ausführungsform der Erfindung,
- Fig. 2 ein Diagramm zur Erläuterung der Pumpzyklen,
- Fig. 3 eine weitere Ausführungsform der Erfindung in einer Abbildung ähnlich der nach Fig. 1,
- Fig. 4 ein Diagramm zur Erläuterung der Pumpzyklen der Anordnung nach Fig. 3, und
- Fig. 5 eine dritte bevorzugte Ausführungsform der Erfindung.

In der nachfolgenden Beschreibung werden für gleiche und gleich wirkende Teile dieselben Bezugsziffern verwendet.

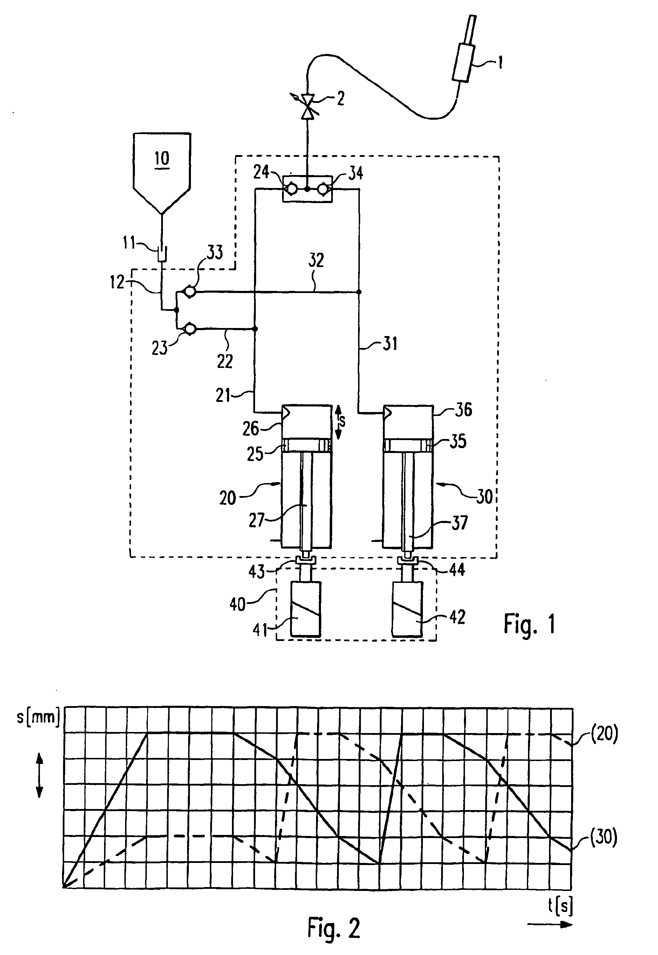

Bei der in Fig. 1 gezeigten Ausführungsform sind eine erste Pumpe 20 und eine zweite Pumpe 30 vorgesehen, die jeweils einen Kolben 25; 35, einen Zylinder 26; 36, eine Kolbenstange 27; 37 und eine Pumpenleitung 21 bzw. 31 aufweisen, die über Ausstoßventile 24 bzw. 34 und ein Klemmventil 2 mit einem chirurgischen Instrument 1 verbunden sind.

Die Pumpenleitungen 21 bzw. 31 sind über Ansaugleitungen 22 bzw. 32 und Ansaugventile 23 bzw. 33 mit einer Ansaugleitung 12 verbunden, die über eine lösbare Kupplung 11 mit einer Quelle 10 verbindbar ist.

Die Kolbenstangen 27 bzw. 37 sind über eine erste Kupplung 43 bzw. eine zweite Kupplung 44 mit einem ersten Motor 41 bzw. zweiten Motor 42 lösbar verbunden. Die in Fig. 1 mit einer unterbrochenen Linie umrissenen Teile sind als "disposable unit" ausgebildet, die beim Hersteller sterilisiert und steril verpackt wird und so in den Handel bzw. zum Anwender gelangt.

Die Antriebsmotoren 41, 42 sind mit einer (nicht gezeigten) Steuerung verbunden, welche die Antriebsmotoren 41, 42 derart ansteuert, dass die Kolben 25 bzw. 35 einen Weg s zurücklegen, dessen zeitlicher Verlauf in Fig. 2 dargestellt ist. Wie in Fig. 2 gezeigt, sind hierbei die Ansaugzyklen, bei denen das Maß s steigt, in Fig. 2 also s nach oben hin zunimmt, sehr viel kürzer als die Ausstoßzyklen (bei denen die Kurven in Fig. 2 fallen). Weiterhin überlappen sich die Ausstoßzyklen derart, dass die Summe der Volumina (definiert durch die Kolben 25 und 35 sowie die Zylinder 26 bzw. 36) pro Zeiteinheit unvermindert gleich bleibt, so dass ein konstanter Ausstrom an Flüssigkeit aus der Quelle 10 zum chirurgischen Instrument 1 gewährleistet ist. Als übliche Werte des Druckes bei Anwendung in der Wasserstrahlchirurgie, können ca. 20 bis 100 bar angegeben werden. Bevorzugte Hubvolumina der Pumpen 20, 30 liegen zwischen 2 und 100 ml pro Hub.

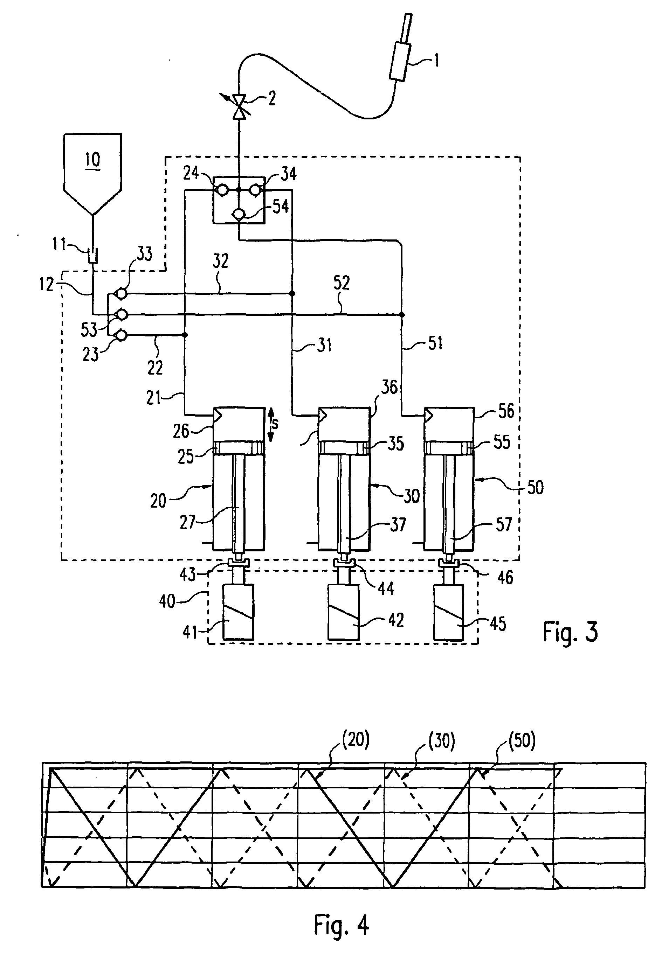

Die in Fig. 3 gezeigte Ausführungsform der Erfindung unterscheidet sich von der nach Fig. 1 in erster Linie dadurch, dass eine dritte Pumpe 50 mit einer dritten Pumpenleitung 51, einer dritten Ansaugleitung 52, einem dritten Ansaugventil 53, einem dritten Ausstoßventil 54, einem dritten Kolben 55, einem dritten Zylinder 56 und einer dritten Kolbenstange 57 sowie ein dritter Motor 45 und eine dritte Kupplung 46 vorgesehen sind, die mit der dritten Kolbenstange 57 verbunden ist.

Weiterhin kann bei dieser Ausführungsform der Erfindung die (hier nicht gezeigte) Steuereinrichtung für die Motoren 41, 42 und 45 derart ausgebildet sein, dass die Ansaugzyklen genauso lang sind wie die Ausstoßzyklen, wobei die drei Zyklen derart überlappend angeordnet sind, dass wiederum eine gleichmäßige (Pumpleistung), also Pumpvolumen pro Zeiteinheit gewährleistet ist. Dies ist in Fig. 4 schematisiert angedeutet, wobei die Kurvenverläufe für die einzelnen Pumpen 20, 30 und 50 mit diesen Bezugsziffern bezeichnet sind.

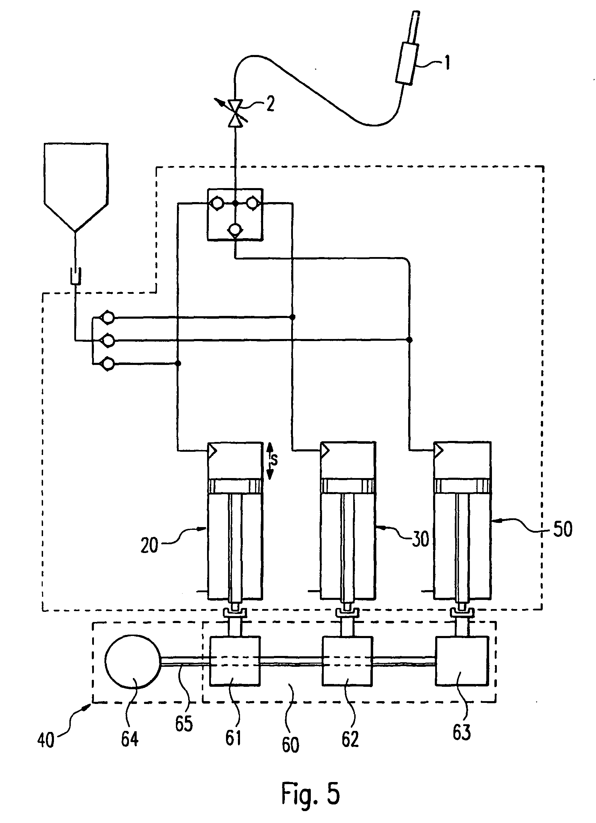

Die weitere, in Fig. 5 gezeigte Ausführungsform der Erfindung entspricht der nach Fig. 3, wobei jedoch keine gesonderten Pumpmotoren 41, 42 und 45 vorgesehen sind. Vielmehr umfasst die Antriebseinrichtung 40 einen einzigen Motor 64, dessen Welle 65 mit einer Getriebeanordnung 60 verbunden ist, das drei Getriebe 61, 62 und 63 umfasst, die jeweils eine Pumpe 20, 30 bzw. 50 antreiben. Die Getriebe 61, 62 und 63 können beispielsweise als Kurvenscheibengetriebe ausgebildet sein, so dass die Betätigungszyklen ähnlich denen nach Fig. 4 ausführbar sind. Selbstverständlich ist es auch möglich, eine derartig ausgestaltete Antriebseinrichtung 40 bei der Ausführungsform nach Fig. 1 zu verwenden.

### Bezugszeichenliste

- 1: Chirurgisches Instrument
- 2: Klemmventil
- 10: Quelle
- 11: Kupplung
- 12: Ansaugleitung
- 20: 1. Pumpe
- 21: 1. Pumpenleitung
- 22: 1. Ansaugleitung
- 23: 1. Ansaugventil
- 24: 1. Ausstoßventil
- 25: 1. Kolben
- 26: 1. Zylinder
- 27: 1. Kolbenstange
- 30: 2. Pumpe
- 31: 2. Pumpenleitung
- 32: 2. Ansaugleitung
- 33: 2. Ansaugventil
- 34: 2. Ausstoßventil
- 35: 2. Kolben
- 36: 2. Zylinder
- 37: 2. Kolbenstange
- 40: Antriebseinrichtung
- 41: 1. Motor
- 42: 2. Motor
- 43: 1. Kupplung
- 44: 2. Kupplung
- 45: 3. Motor
- 46: 3. Kupplung
- 50: 3. Pumpe
- 51: 3. Pumpenleitung
- 52: 3. Ansaugleitung
- 53: 3. Ansaugventil
- 54: 3. Ausstoßventil
- 55: 3. Kolben
- 56: 3. Zylinder
- 57: 3. Kolbenstange
- 60: Getriebe
- 61: 1. Teilgetriebe
- 62: 2. Teilgetriebe
- 63: 3. Teilgetriebe
- 64: Motor
- 65: Welle

## Patentansprüche

1. Fördereinrichtung für sterile Fluide zum Fördern eines sterilen Fluids von einem Reservoir oder dergleichen Quelle (10) zu einem chirurgischen Instrument für die Wasserstrahlchirurgie, umfassend,
eine Kolbenpumpe oder dergleichen volumetrisch fördernde Pumpe (20, 30) mit einem Ansaugzyklus zum Ansaugen des Fluids und einem Ausstoßzyklus zum Ausstoßen des Fluids,
Leitungs- und Ventileinrichtungen (12; 21 bis 24; 31 bis 34) zum Verbinden der Pumpe (20, 30) mit der Quelle (10) und dem Verbraucher (1) und
Antriebseinrichtungen (40) zum Antreiben der Pumpe (20, 30),
wobei die Pumpe mindestens eine erste und eine zweite Kolben-/Zylinder-Einheit oder dergleichen erste und zweite Pumpkammern (25, 26, 35, 36) aufweist,
wobei die Antriebseinrichtungen (40) derart ausgebildet und mit der Pumpe (20, 30) verbunden sind,
dass der Ansaugzyklus kürzer ist als der Ausstoßzyklus,
dass sich die Ausstoßzyklen überlappen,
dass die Pumpe (20, 30, 50) mit der Antriebseinrichtung (40) lösbar verbunden ist, und dass die Pumpe (20, 30, 50) und die Leitungseinrichtungen (12; 21, 22; 31, 32; 51, 52) zusammen mit den Ventileinrichtungen (23, 24; 33, 34; 53, 54) als "disposable unit" ausgebildet ist.

2. Fördereinrichtung für sterile Fluide zum Fördern eines sterilen Fluids von einem Reservoir oder dergleichen Quelle (10) zu einem chirurgischen Instrument für die Wasserstrahlchirurgie, umfassend,
eine Kolbenpumpe oder dergleichen volumetrisch fördernde Pumpe (20, 30) mit einem Ansaugzyklus zum Ansaugen des Fluids und einem Ausstoßzyklus zum Ausstoßen des Fluids,
Leitungs- und Ventileinrichtungen (12; 21 bis 24; 31 bis 34) zum Verbinden der Pumpe (20, 30) mit der Quelle (10) und dem Verbraucher (1) und
Antriebseinrichtungen (40) zum Antreiben der Pumpe (20, 30),
wobei die Pumpe (20, 30, 50) mindestens drei Pumpkammern (25, 26, 35, 36) aufweist und die Antriebseinrichtung (40) derart ausgebildet ist,
dass die Ansaug- und die Ausstoßzyklen der Pumpkammern einander überlappen, dass sich die Ausstoßzyklen überlappen,
dass die Pumpe (20, 30, 50) mit der Antriebseinrichtung (40) lösbar verbunden ist, und dass die Pumpe (20, 30, 50) und die Leitungseinrichtungen (12; 21, 22; 31, 32; 51, 52) zusammen mit den Ventileinrichtungen (23, 24; 33, 34; 53, 54) als "disposable unit" ausgebildet ist.

3. Fördereinrichtung nach Anspruch 1,
**dadurch gekennzeichnet, dass**
die mindestens erste und zweite Kolben-/Zylinder-Einheit oder dergleichen erste und zweite Pumpkammern (25, 26; 35, 36) im Gegentakt derart steuerbar sind, dass der Ansaugzyklus in der ersten Pumpkammer (25, 26) kürzer ist als der Ausstoßzyklus in der zweiten Pumpkammer (35, 36) und umgekehrt.

4. Fördereinrichtung nach einem der vorhergehenden Ansprüche,
**dadurch gekennzeichnet, dass**
die Antriebseinrichtung (40) derart ausgebildet ist, dass das Fluid dem Verbraucher (1) mit einem im Wesentlichen konstanten Druck zugeführt wird.

5. Fördereinrichtung nach einem der vorhergehenden Ansprüche,
**dadurch gekennzeichnet, dass**
die Antriebseinrichtung (40) für jede Pumpkammer (25, 26; 34, 36; 54, 56) einen gesonderten steuerbaren Antriebsmotor (41, 42, 45) umfasst.

6. Fördereinrichtung nach einem der Ansprüche 1 bis 5,
**dadurch gekennzeichnet, dass**
die Antriebseinrichtung (40) einen einzigen steuerbaren Antriebsmotor (64) sowie Getriebeeinrichtungen (60) mit einem mit dem Antriebsmotor (64) verbundenen Getriebeeingang und einem Getriebeausgang für jede Pumpenkammer (25, 26; 35, 36; 55, 56) aufweist.

## Claims

1. Transport device for sterile fluid for transporting a sterile fluid from a reservoir or similar source (10) to a surgical instrument for water jet surgery, comprising
a piston pump or similar volumetric pump (20, 30) with a suction cycle for sucking in the fluid and an output cycle for expelling the fluid,
control and valve devices (12; 21 to 24; 31 to 34) for connecting the pump (20, 30) to the source (10) and the consumer (1) and
drive devices (40) for driving the pump (20, 30),
wherein the pump has at least a first and a second piston/cylinder unit or similar first and second pump chambers (25, 26, 35, 36),
wherein the drive devices (40) are configured and connected to the pump (20, 30) in such a way
that the suction cycle is shorter than the output cycle,
that the output cycles overlap,
that the pump (20, 30, 50) is detachably connected to the drive device (40), and that the pump (20, 30, 50) and the control devices (12; 21, 22; 31, 32; 51, 52) together with the valve devices (23, 24; 33, 34; 53, 54) are configured as a disposable unit.

2. Transport device for sterile fluid for transporting a sterile fluid from a reservoir or similar source (10) to a surgical instrument for water jet surgery, comprising
a piston pump or similar volumetric pump (20, 30) with a suction cycle for sucking in the fluid and an output cycle for expelling the fluid,
control and valve devices (12; 21 to 24; 31 to 34) for connecting the pump (20, 30) to the source (10) and the consumer (1) and
drive devices (40) for driving the pump (20, 30),
whereby the pump (20, 30, 50) has at least three pump chambers (25, 26, 35, 36) and the drive device (40) is configured in such a way that the suction and the output cycles of the pump chambers overlap into each other,
that the output cycles overlap,
that the pump (20, 30, 50) is detachably connected to the drive device (40), and that the pump (20, 30, 50) and the control devices (12; 21, 22; 31, 32; 51, 52) together with the valve devices (23, 24; 33, 34; 53, 54) are configured as a disposable unit.

3. Transport device according to Claim 1,
**characterised in that**
the at least first and second piston/cylinder unit or similar first and second pump chambers (25, 26, 35, 36) can be controlled in a push-pull manner, in such a way that the suction cycle in the first pump chamber (25, 26) is shorter than the output cycle in the second pump chamber (35, 36) and vice versa.

4. Transport device according to one of the previous claims,
**characterised in that**
the drive device (40) is configured in such a way that the fluid is transported to the consumer (1) under a substantially constant pressure.

5. Transport device according to one of the previous claims,
**characterised in that**
the drive device (40) for each pump chamber (25, 26; 35, 36; 55, 56) comprises a separate controllable drive motor (41, 42, 45).

6. Transport device according to one of the Claims 1 to 5,
**characterised in that**
the drive device (40) has a single controllable drive motor (64) as well as gear devices (60) with a gear input connected to the drive motor (64) and a gear output for each pump chamber (25, 26; 35, 36; 55, 56).

## Revendications

1. Dispositif de transport pour des fluides stériles, pour le transport d'un fluide stérile d'un réservoir ou d'une source similaire (10) vers un instrument chirurgical pour la chirurgie à jet d'eau, comprenant,
une pompe à piston ou une pompe à refoulement volumétrique similaire (20, 30) avec un cycle d'aspiration pour l'aspiration du fluide et un cycle de refoulement pour le refoulement du fluide,
des dispositifs de conduits et de vannes (12 ; 21 à 24 ; 31 à 34) pour le raccordement de la pompe (20, 30) à la source (10) et au récepteur (1), et
des dispositifs d'entraînement (40) pour l'entraînement de la pompe (20, 30),
où la pompe comporte au moins une première et une deuxième unité piston/cylindre ou une première et une deuxième chambre de pompe similaires (25, 26, 35, 36),
où les dispositifs d'entraînement (40) sont réalisés et reliés à la pompe (20, 30) de telle manière,
que le cycle d'aspiration est plus court que le cycle de refoulement,
que les cycles de refoulement se chevauchent,
que la pompe (20, 30, 50) est reliée de manière amovible au dispositif d'entraînement (40),
et que la pompe (20, 30, 50) et les dispositifs de conduits (12 ; 21, 22 ; 31, 32 ; 51, 52) ainsi que les dispositifs de vannes (23, 24 ; 33, 34 ; 53, 54) sont réalisés comme « unité jetable ».

2. Dispositif de transport pour des fluides stériles, pour le transport d'un fluide stérile d'un réservoir ou d'une source similaire (10) vers un instrument chirurgical pour la chirurgie à jet d'eau, comprenant,
une pompe à piston ou une pompe à refoulement volumétrique similaire (20, 30) avec un cycle d'aspiration pour l'aspiration du fluide et un cycle de refoulement pour le refoulement du fluide,
des dispositifs de conduits et de vannes (12 ; 21 à 24 ; 31 à 34) pour le raccordement de la pompe (20, 30) à la source (10) et au récepteur (1), et
des dispositifs d'entraînement (40) pour l'entraînement de la pompe (20, 30),
où la pompe (20, 30, 50) comporte au moins trois chambres de pompe (25, 26, 35, 36), et où le dispositif d'entraînement (40) est réalisé de telle manière
que les cycles d'aspiration et de refoulement des chambres de pompe se chevauchent l'un l'autre,
que les cycles de refoulement se chevauchent,
que la pompe (20, 30, 50) est reliée de manière amovible au dispositif d'entraînement (40),
et que la pompe (20, 30, 50) et les dispositifs de conduits (12 ; 21, 22 ; 31, 32 ; 51, 52) ainsi que les dispositifs de vannes (23, 24 ; 33, 34 ; 53, 54) sont réalisés comme « unité jetable ».

3. Dispositif de transport selon la revendication 1,
**caractérisé**
**en ce que** la ou les premières et deuxièmes unités piston/cylindre ou la première et la deuxième chambres de pompe similaires (25, 26 ; 35, 36) sont commandables en opposition de telle manière que le cycle d'aspiration de la première chambre de pompe (25, 26) soit plus court que le cycle de refoulement de la deuxième chambre de pompe (35, 36), et inversement.

4. Dispositif de transport selon l'une des revendications précédentes,
**caractérisé**
**en ce que** le dispositif d'entraînement (40) est réalisé de telle manière que le fluide est conduit vers le récepteur (1) avec une pression sensiblement constante.

5. Dispositif de transport selon l'une des revendications précédentes,
**caractérisé**
**en ce que** le dispositif d'entraînement (40) pour chaque chambre de pompe (25, 26 ; 34, 36 ; 54, 56) comprend un moteur d'entraînement (41, 42, 45) commandable séparément.

6. Dispositif de transport selon l'une des revendications 1 à 5,
**caractérisé**
**en ce que** le dispositif d'entraînement (40) comporte un seul moteur d'entraînement (64) commandable ainsi que des dispositifs d'engrenage (60) avec une entrée d'engrenage raccordée au moteur d'entraînement (64) et une sortie d'engrenage pour chaque chambre de pompe (25, 26 ; 35, 36 ; 55, 56).
